# EUROPEAN PATENT SPECIFICATION

(11) **EP 2 052 735 B1**
(45) Date of publication and mention of the grant of the patent: **11.05.2016**
(21) Application number: 07020633.9
(22) Date of filing: 22.10.2007
(51) Int. Cl.: A61K 38/05, A61K 9/48

(54) **Pharmaceutical composition and kit comprising anserine for rapidly reducing uric acid in blood**
Pharmazeutische Zusammensetzung und Kit enthaltend Anserin zur schnellen Reduzierung des Harnsäureanteils im Blut
Composition pharmaceutique et ensemble comprenant anserine pour réduire rapidement l'acide urique dans le sang

(43) Date of publication of application: 29.04.2009
(73) Proprietor: Lytone Enterprise, Inc., New Taipei City 221 (TW)
(72) Inventor: Chang, William, T.H., Taiwan, R.O.C. (TW); Chen, Hsi, Ying, Taiwan, R.O.C. (TW); Wang, Yi Chieh, Taiwan, R.O.C. (TW)
(74) Representative: TBK

(56) References cited:
- EP-A- 0 710 485
- EP-A- 1 586 332
- US-A1- 2004 242 491

## Description

### Field of the Invention

The present invention relates to the administration of anserine in an amount of about 100 mg to about 600 mg so as to rapidly reduce uric acid level in blood in a subject.

### Background of the Invention

Uric acid is the final product of purine metabolism in human beings. The uric acid level in blood is a function of the balance between the breakdown of purines and the rate of uric acid excretion. For years, hyperuricemia has been associated with or thought to be the same as gout, but uric acid has now been identified as a marker for a number of metabolic and hemodynamic abnormalities. A high uric acid level in blood induces gout, and is normally related to symptoms such as muscle spasm, localized swelling, inflammation, joint pains, muscle fatigue, feelings of stress, and myocardial infarction.

Many commercialized drugs have been used to treat gout, for example, Benzbromarone (URINORM), Probenecid, Allopurinol, Bucolome, Cinchophan and Colchicine. These drugs work by, for example, inhibiting the formation of uric acid, removing excessive uric acid, acting on the kidneys so as to help to eliminate uric acid, inhibiting the activity of xanthine oxidase for converting xanthine into uric acid, and accelerating the excretion of uric acid. However, these drugs are known to have hepatotoxicity, and cause a number of side effects, such as urinary calculus, gastrointestinal obstruction, jaundice, and anemia. These drugs are not reported to be capable of rapidly reducing the uric acid level in blood in a subject.

It is known that some dipeptides are useful in lowering the uric acid level in blood. For example, ROC (Taiwan) Patent No. I280136 (ROC (Taiwan) Patent Application No. 092114798) discloses a composition for reducing uric acid in a subject, which comprises an effective amount of one or more dipeptides consisting of histidine or functional equivalent thereof and alanine or functional equivalent thereof. Exemplary dipeptides mentioned in the ROC patent include, carnosine, anserine, carcinine, and ophidine. It only teaches that the composition is administered orally to provide an amount of about 8 to about 50 mg, preferably about 10 mg to about 45 mg, of the dipeptide(s) per day, but does not disclose or suggest any route that may rapidly reduce uric acid level in blood in a subject by administering dipeptide(s).

Anserine (β-alanyl-1-methyl-L-histidine) is a highly stable dipeptide, which has a pK value of approximately 7.1, and remains intact under low pH (<3.0). Anserine naturally exists in the skeletal muscle tissue and brain tissue of vertebrates, and is formed by a peptide bond between β-alanine and 1-methyl-L-histidine (see Tolkachevskaya, NF. 1929. Z Physiol Chem. 185:28-32.). In 1938, it was reported that anserine is an ideal physiological acid-base buffering agent. Due to its physiological acid-base buffering capability, anserine is effective in eliminating the lipid peroxidation promoted by changing the pH values of biological systems. For a subject with acidemia who suffers from gout, the administration of anserine acting as an acid-base buffering agent helps to adjust the pH value of blood, and is advantageous in lowering the uric acid level in blood. It should be understood that 40% of the buffering capability of fast-twitch muscle fibers is contributed by dipeptides in a mobile phase, while the remaining proportion of the buffering capability of fast-twitch muscle fibers is contributed by muscle proteins which are essentially in a fixed phase (see Bate-Smith, EC. 1938, "The buffering of muscle in rigour: protein, phosphate and carnosine.," J Physiol. 92:336-43.). This shows that in highly structured muscle cells, local acid-base gradient is composed of buffering assistance of the mobile phase and buffering suppression of the fixed phase.

Moreover, anserine has various antioxidant properties, and is useful as a free radical scavenger or a metal chelating agent. Subjects suffering from inflammation for a long period of time normally have a higher *in vivo* free radical concentration. The administration of anserine acting as a free radical scavenger is advantageous to eliminate free radicals.

Anserine is also effective in inhibiting the lipid peroxidation promoted by iron, heamoglobin, lipid peroxidase, and singlet oxygen (see Babizhayev MA et. al., 1994, "L-carnosine (beta-alanyl-L-histidine) and carcinine (beta-alanylhistamine) act as natural antioxidants with hydroxyl-radical-scavenging and lipid-peroxidase activities," Biochem J. 304 (Pt 2): 509-516, and Kohen R. et. al., 1988, "Antioxidant activity of cannosine, homocarnosine, and anserine present in muscle and brain," Proc Natl Acad Sci USA. 85(9):3175-3179.).

In addition, anserine can ameliorate *in vivo* oxidative stress caused by fatigue, and enhance oxygen radical absorbance capacity in organisms. Anserine may also act as a precursor of transmitters in central histaminergic nervous system. Through the metabolism of histidine contained in anserine, anserine can effectively adjust the concentration of histamine in central nervous system, lower the concentration of cortisol in blood, and cause a relief of fatigue (see Aoi W. et al., 2006, "Exercise and functional foods.," Nutr J. 5: 15-22, and U.S. Patent No. 6,680,294.

However, commercially available drugs for treating gout comprising anserine are not reported to have an efficacy of rapidly reducing uric acid level in blood in a subject.

There is still a need to develop a new drug which can rapidly reduce uric acid level in blood in a subject, thereby treating gout and alleviating the symptoms associated with a high uric acid level in blood.

EP 0 710 485 A discloses a composition stimulating hemopoietic function, or preventing or alleviating medical symptoms caused by the decrease of the number of erythrocytes. The composition of this document comprises at least one imidazole compound, for example, anserine.

EP 1 586 332 relates to the combined use of carnosinase inhibitor with L-carnosines, which are useful in the treatment or prevention of various diseases, the improvement of health conditions, the improvement of exercise ability, the improvement of skin health, the prevention of the side effects in mammals of drinking alcohol, and the like.

US 2004/242491 A1 discloses the administration of one or more dipeptides preferably comprising up to 95% w/w anserine in an amount from about 8 mg to about 50 mg per day in order to reduce uric acid in a patient, to treat gout, and to reduce symptoms related to high levels of uric acid in a patient, for example, muscle spasm, localized swelling, inflammation, joint pains, muscle fatigue, feelings of stress and myocardial infarction.

### Summary of the Invention

The inventors have surprisingly discovered that the administration of anserine in an amount of about 100 mg to about 600 mg per dose can rapidly reduce the uric acid level in blood in the subject.

Therefore, the present invention relates to a pharmaceutical composition for rapidly reducing the uric acid level in blood in a subject, which comprises anserine in an amount of about 100 mg to about 600 mg for use as defined in claim 1.

In addition, the present invention relates to the use of anserine in the manufacture of a medicament for rapidly reducing the uric acid level in blood in a subject, wherein the medicament comprises anserine in an amount of about 100 mg to about 600 mg as defined in claim 4.

### Brief Description Of Drawings

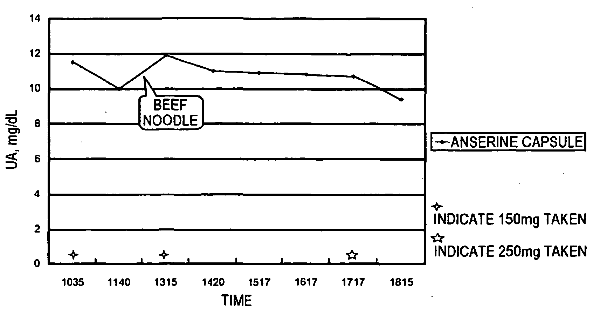

Figure 1 shows the influence of orally administered anserine on the uric acid level in blood.

### Detailed Description of the Invention

In the first aspect, the present invention provides a pharmaceutical composition for rapidly reducing the uric acid level in blood in a subject, which comprises anserine in an amount of about 100 mg to about 600 mg for use as defined in claim 1. The pharmaceutical composition of the invention can rapidly reduce the uric acid level in blood in a subject, thereby treating gout and/or ameliorating the symptoms associated with a high uric acid level in blood.

In the second aspect, the present invention provides a use of anserine in the manufacture of a medicament capable of rapidly reducing the uric acid level in blood in a subject, wherein the medicament comprises anserine in an amount of about 100 mg to about 600 mg as defined in claim 4. The medicament can rapidly reduce the uric acid level in blood in a subject, thereby treating gout and/or ameliorating the symptoms associated with a high uric acid level in blood.

Anserine may be naturally occurring or synthesized. In the present invention, anserine is administered to a subject in an amount of about 100 mg to about 600 mg, preferably from about 100 mg to about 500 mg, more preferably from about 150 mg to 250 mg, per dose, in order to achieve an efficacy of rapidly reducing the uric acid level in blood in the subject.

In the subject application, the term "rapidly reducing the uric acid level in blood" means that the reduced amount of the uric acid level in blood in a subject is about 0.5 to about 4 mg/dL, within one hour of the administration of anserine. The term "a subject" refers to a mammal, preferably a human. In addition, the term "symptoms related to a high level of uric acid" refers to, for example, muscle spasm, localized swelling, inflammation, joint pains, muscle fatigue, feelings of stress, myocardial infarction, or the combination thereof.

The pharmaceutical composition and medicament of the invention can be prepared by any conventional methods known in the art. In addition to the active ingredient, anserine, the pharmaceutical composition and medicament of the invention may comprise a pharmaceutically or physiologically acceptable excipient. Suitable pharmaceutically acceptable excipients include, but are not limited to, dextrin-maltose, lactose, sucrose, mannitol, starch, microcrystalline cellulose, carboxymethyl cellulose, talc, and magnesium stearate.

The pharmaceutical composition and medicament of the invention may be formulated in appropriate dosage forms, such as tablets, powder, granules, capsules, liquid, and suspension, for various administration routes. The pharmaceutical composition and medicament of the invention may be administered via any suitable routes, preferably via oral or parenteral route.

### Examples

The invention will become apparent with reference to the following examples, which are purely exemplary, and should not be taken to limit the scope of the invention as described in the claims.

### Example 1

Anserine (GL Biochem Ltd., Shanghai, China) and dextrin-maltose were filled into a gelatin capsule (total weight 400 mg), and the capsule comprised 50 mg of anserine. A subject suffering from gout and having a high level of uric acid in blood took three capsules (a total 150 mg of anserine) two times a day and five capsules (a total 250 mg of anserine) once in the same day, so the total dosage of anserine taken by the subject daily was 550 mg. The uric acid level in blood was monitored by UroSpeed, a rapid sero-uric acid test strip (Apex Biotechnology Co., Inc. Hsinchu, Taiwan, ROC). The results are shown in Table 1, and further drawn in Figure 1.

**Table 1: Decrease in the uric acid level in blood one hour after anserine is orally administered**

| Time | Anserine dosage | Uric acid level in blood (mg/dL) | Decrease in uric acid level in blood 1 hour after anserine is orally administered | |
|---|---|---|---|---|
| | | | (mg/dL) | (%) |
| 10:35 | 150 mg | 11.5 | 1.5 | 13.04% |
| 11:40 | - | 10.0 | | |
| 13:15 | 150 mg | 11.9 | 0.9 | 7.56% |
| 14:20 | - | 11.0 | | |
| 17:17 | 250 mg | 10.7 | 1.3 | 12.15% |
| 18:15 | - | 9.4 | | |

As shown in Table 1 and Figure 1, the range of decrease in the level of uric acid in blood is from 0.9 mg/dL to 1.5 mg/dL one hour after the regimen of the capsule comprising anserine. One hour after the regimen of 150 mg of anserine at 10:35, the level of uric acid in blood decreased by 1.5 mg/dL. After the subject ate beef noodle at noon, the level of uric acid in the subject's blood increased to 11.9 mg/dL. One hour after regimen of 150 mg of anserine at 13:15, the level of uric acid in blood of the subject decreased by 0.9 mg/dL. One hour after consuming 250 mg of anserine at 17:17, the uric acid level in blood of the subject decreased by 1.3 mg/dL. The data given in Table 1 reveals that the administration of anserine can rapidly reduces the uric acid level in blood in a subject within one hour.

### Example 2

Fifteen voluntary adult subjects (nine males and six females 28 to 53 years old) having chronic hyperuricemia were tested in this oral administration test. These subjects stopped their regular medication for at least 3 days. The average uric acid level in blood of these subjects became greater than 9.3 mg/dl. These subjects were randomly separated in five groups, each comprising three subjects, to take anserine in the following dosages: 50 mg, 100 mg, 150 mg, 200 mg, and 250 mg, respectively. After one hour, the uric acid levels in the blood of the subjects were measured. It was found that the uric acid level in their blood decreased by 1.1 mg/dL to 1.3 mg/dL. Among the five groups, the decrease in the uric acid level in blood in the group taking 150 mg of anserine is the most significant one. The results are shown in Table 2.

**Table 2: Influence on uric acid level in blood 1 hour after various dosages of anserine are orally administered**

| Anserine (mg/day) | Uric acid level in blood (mg/dL) | | Decrease in uric acid in blood 1 hour after anserine is orally administered | |
|---|---|---|---|---|
| | 0 hr | 1 her | (mg/dL) | (%) |
| 50 | 9.1±1.3 | 7.9±1.0 | 1.3±0.5 | 13.7 |
| 100 | 8.8±1.2 | 7.6±1.4 | 1.2±0.5 | 13.9 |
| 150 | 8.5±0.7 | 7.3±0.9 | 1.2±0.2 | 14.6 |
| 200 | 10.1±1.4 | 8.8±1.4 | 1.3±0.3 | 13.1 |
| 250 | 9.9±0.9 | 8.8±1.0 | 1.1±0.3 | 11.5 |

## Claims

1. A pharmaceutical composition comprising anserine in an amount of 100 mg to 600 mg per dose for use in the treatment of gout and/or to alleviate symptoms associated with a high uric acid level in blood.

2. The composition for use as defined in Claim 1, wherein anserine is comprised in an amount of 100 mg to 500 mg.

3. The composition for use as defined in Claim 2, wherein anserine is comprised in an amount of 150 mg to 250 mg.

4. The composition for use as defined in Claim 1, wherein the symptom is selected from muscle spasm, localized swelling, inflammation, joint pains, muscle fatigue, feelings of stress, myocardial infarction, and the combination thereof.

5. Use of anserine in the manufacture of a medicament for in the treatment of gout and/or alleviating symptoms associated with a high uric acid level in blood, wherein the medicament is for the administration of anserine in an amount of 100 mg to 600 mg per dose.

6. The use according to Claim 5, wherein the medicament is for the administration of anserine in an amount of 100 mg to 500 mg per dose.

7. The use according to Claim 6, wherein the medicament is for the administration of anserine in an amount of 150 mg to 250 mg per dose.

8. The use according to Claim 5, wherein the symptom is selected from muscle spasm, localized swelling, inflammation, joint pains, muscle fatigue, feelings of stress, myocardial infarction, and the combination thereof.

## Patentansprüche

1. Pharmazeutische Zusammensetzung, die Anserin in einer Menge von 100 mg bis 600 mg pro Dosis umfasst, für die Verwendung in der Behandlung von Gicht und/oder um Symptome zu lindern, die mit einem hohen Harnsäurespiegel im Blut verbunden sind.

2. Zusammensetzung für die Verwendung wie in Anspruch 1 definiert, wobei Anserin in einer Menge von 100 mg bis 500 mg umfasst ist.

3. Zusammensetzung für die Verwendung wie in Anspruch 2 definiert, wobei Anserin in einer Menge von 150 mg bis 250 mg umfasst ist.

4. Zusammensetzung für die Verwendung wie in Anspruch 1 definiert, wobei das Symptom aus Muskelkrampf, lokaler Schwellung, Entzündung, Gelenkschmerzen, Muskelermüdung, Beanspruchungsempfindungen, Myokardinfarkt und der Kombination davon ausgewählt ist.

5. Verwendung von Anserin bei der Herstellung eines Medikaments für die Behandlung von Gicht und/oder für die Linderung von Symptomen, die mit einem hohen Harnsäurespiegel im Blut verbunden sind, wobei das Medikament für die Verabreichung von Anserin in einer Menge von 100 mg bis 600 mg pro Dosis ist.

6. Verwendung nach Anspruch 5, wobei das Medikament für die Verabreichung von Anserin in einer Menge von 100 mg bis 500 mg pro Dosis ist.

7. Verwendung nach Anspruch 6, wobei das Medikament für die Verabreichung von Anserin in einer Menge von 150 mg bis 250 mg pro Dosis ist.

8. Verwendung nach Anspruch 5, wobei das Symptom aus Muskelkrampf, lokaler Schwellung, Entzündung, Gelenkschmerzen, Muskelermüdung, Beanspruchungsempfindungen, Myokardinfarkt und der Kombination davon ausgewählt ist.

## Revendications

1. Composition pharmaceutique comprenant de l'ansérine en une quantité de 100 mg à 600 mg par dose destinée à une utilisation dans le traitement de la goutte et/ou pour soulager des symptômes associés à un niveau élevé d'acide urique dans le sang.

2. Composition destinée à une utilisation telle que définie dans la revendication 1, dans laquelle l'ansérine est comprise dans une quantité de 100 mg à 500 mg.

3. Composition destinée à une utilisation telle que définie dans la revendication 2, dans laquelle l'ansérine est comprise dans une quantité de 100 mg à 250 mg.

4. Composition destinée à une utilisation telle que définie dans la revendication 1, dans laquelle le symptôme est choisi parmi un spasme musculaire, un gonflement localisé, une inflammation, des douleurs articulaires, une fatigue musculaire, des sentiments de stress, un infarctus du myocarde, et la combinaison de ceux-ci.

5. Utilisation d'ansérine dans la fabrication d'un médicament pour le traitement de la goutte et/ou pour soulager des symptômes associés à un niveau élevé d'acide urique dans le sang, dans laquelle le médicament est pour l'administration d'ansérine dans une quantité de 100 mg à 600 mg par dose.

6. Utilisation selon la revendication 5, dans laquelle le médicament est pour l'administration d'ansérine dans une quantité de 100 mg à 500 mg par dose.

7. Utilisation selon la revendication 6, dans laquelle le médicament est pour l'administration d'ansérine dans une quantité de 100 mg à 250 mg par dose.

8. Utilisation selon la revendication 5, dans laquelle le symptôme est choisi parmi un spasme musculaire, un gonflement localisé, une inflammation, des douleurs articulaires, une fatigue musculaire, des sentiments de stress, un infarctus du myocarde, et la combinaison de ceux-ci.
